(19)

**Europäisches Patentamt**

**European Patent Office**

**Office européen des brevets**

(11)  **EP 4 763 884 A1**

(12) # EUROPEAN PATENT APPLICATION

(43) Date of publication:
**24.06.2026 Bulletin 2026/26**

(21) Application number: **24221617.4**

(22) Date of filing: **19.12.2024**

(51) International Patent Classification (IPC):
*C08G 18/73* (2006.01)   *C07C 263/10* (2006.01)
*C07C 265/04* (2006.01)

(52) Cooperative Patent Classification (CPC):
**C08G 18/73; C07C 263/10; C07C 265/04**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Covestro Deutschland AG
51373 Leverkusen (DE)**

(72) Inventors:
• **Latorre Martinez, Irene Cristina
40764 Langenfeld (DE)**
• **Pires, Raul
50670 Köln (DE)**
• **Richter, Frank
51373 Leverkusen (DE)**
• **Darmandeh, Heidar
40597 Düsseldorf (DE)**

(74) Representative: **Levpat
c/o Covestro AG
Gebäude K12
51365 Leverkusen (DE)**

(54) **CIS- AND TRANS-2,5-BIS(ISOCYANATOMETHYL) TETRAHYDROFURAN**

(57)    The present invention relates to a 2,5-bis(isocyanatomethyl) tetrahydrofuran consisting of cis-2,5-bis(isocyanatomethyl) tetrahydrofuran and trans-2,5-bis(isocyanatomethyl) tetrahydrofuran in a weight ratio of 1.0:99.0 to 99.9:0.1, a polyisocyanate composition obtainable from the 2,5-bis(isocyanatomethyl) tetrahydrofuran, a method for production of the polyisocyanate composition, a use of the 2,5-bis(isocyanatomethyl) tetrahydrofuran and the polyisocyanate composition, a two-component system comprising the polyisocyanate composition, a one-component system comprising the polyisocyanate composition as well as an article comprising or being coated with the cured product of the two-component system or the one-component system.

**EP 4 763 884 A1**

**Description**

The present invention relates to a 2,5-bis(isocyanatomethyl) tetrahydrofuran consisting of cis-2,5-bis(isocyanatomethyl) tetrahydrofuran and trans-2,5-bis(isocyanatomethyl) tetrahydrofuran in a weight ratio of 1.0:99.0 to 99.9:0.1, a polyisocyanate composition obtainable from the 2,5-bis(isocyanatomethyl) tetrahydrofuran, a method for production of the polyisocyanate composition, a use of the 2,5-bis(isocyanatomethyl) tetrahydrofuran and the polyisocyanate composition, a two-component system comprising the polyisocyanate composition, a one-component system comprising the polyisocyanate composition as well as an article comprising or being coated with the cured product of the two-component system or the one-component system.

The oligomerization or polymerization of isocyanates, especially to form higher molecular weight oligomer mixtures has long been known. The reaction of a relatively small number of isocyanates with one another is referred to as oligomerization. The reaction of a relatively large number of isocyanates is referred to as polymerization. In the context of the present invention, the oligomerization or polymerization of isocyanates described above is referred to collectively as isocyanate modification or modification of isocyanates. All products resulting from such processes are referred to collectively in the present document as polyisocyanate composition or also polyisocyanates for short. The polyisocyanates contain free isocyanate groups, NCO groups for short, which optionally may also have been temporarily deactivated with blocking agents, they are exceptionally high-quality starting materials for the preparation of a multiplicity of polyurethane plastics and coating compositions.

Polyisocyanates employed in coating compositions for high-quality coatings are, in particular, low-monomer derivatives prepared from hexamethylene 1,6-diisocyanate (HDI). Particularly suitable for elastic, highly robust coatings for this purpose are isocyanurate polyisocyanates of HDI, or HDI polyisocyanates containing iminooxadiazinedione and isocyanurate structures, as are described for example in H. J. Laas, R. Halpaap, J. Pedain, J. prakt. Chem. 1994, 336, 185-200. These polyisocyanates generally have isocyanate functionalities ($F_{NCO}$) of 3 or more, where isocyanate functionality refers to the average number of NCO groups per molecule.

There is a steady demand for polyurethane coatings with ever improved properties such as, for example, improved reactivity behaviour, in particular faster and/or even adjustable curing times, compared with coatings of the state of the art which include coatings comprising polyurethanes prepared from HDI, pentamethylene 1,5-diisocyanate (PDI), isophorone diisocyanate (IPDI) or acyclic ether diisocyanates.

GB936370 describes a generic lab synthesis of monomeric isocyanates of furan and tetrahydrofuran by liquid phase phosgenation of the dihydrochloride salt, which is a rather inefficient and complex process on a larger scale. Neither stereoisomers nor further modifications or application tests are described.

WO2011/098272A2 describes mixtures of short-chain renewable diisocyanates with long-chain renewable diisocyanates for preparation of a polyurethane. Among the very long list of short-chain renewable diisocyanates furan based diisocyanates are generally mentioned, but neither application examples nor preferences are described.

WO 2021/180709 discloses ether isocyanates and kinetic measurements investigating reactivity of said isocyanates towards NCO-reactive compounds. However, none of the disclosed isocyanate compounds is a cyclic compound, nor are cis and trans isomers of cyclic compounds investigated.

It was thus an object of the present invention to provide a diisocyanate that exhibits improved curing behaviour, in particular faster and/or adjustable curing, when reacted with NCO-reactive compounds and resulting polyisocyanate compositions.

This object has surprisingly been achieved by a 2,5-bis(isocyanatomethyl) tetrahydrofuran consisting of cis-2,5-bis(isocyanatomethyl) tetrahydrofuran and trans-2,5-bis(isocyanatomethyl) tetrahydrofuran in a weight ratio of 1.0:99.0 to 99.9:0.1.

The invention is thus a diisocyanate which consists of the cis and trans isomers of 2,5-bis(isocyanatomethyl) tetrahydrofuran.

The references to "comprising", "containing", etc. preferably denote "substantially consisting of" and very particularly preferably denote "consisting of".

In the present invention, any numerical range recited herein is intended to include all sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

According to this invention, the cis and trans isomers of 2,5-bis(isocyanatomethyl) tetrahydrofuran are jointly referred to as "TEFUDI" and each individually also referred to as "cis-TEFUDI" and "trans-TEFUDI". For sake of clarity, cis-TEFUDI corresponds to the (2R,5S) and (2S,5R) configuration and trans-TEFUDI corresponds to the (2R,5R) and (2S,5S) configuration.

As shown in the experimental section, cis-TEFUDI seems to exhibit higher reactivity towards NCO-reactive compounds and thus allows for faster curing than trans-TEFUDI. It is thus often desirable to have at least 50% by weight of cis-TEFUDI present in the inventive 2,5-bis(isocyanatomethyl) tetrahydrofuran. Thus, according to one embodiment of the invention, the 2,5-bis(isocyanatomethyl) tetrahydrofuran consists of cis-2,5-bis(isocyanatomethyl) tetrahydrofuran and trans-2,5-bis(isocyanatomethyl) tetrahydrofuran in a weight ratio of 50.0:50.0 to 99.9:0.1, 51.0:49.0 to 99.9:0.1 or 52.0:48.0 to 99.9:0.1, preferably 53.5:46.5 to 99.9:0.1 or 55:45 to 99.9:0.1, more preferably 60.0:40.0 to 99.5:0.5, even more preferably 65.0:35.0 to 99.0: 1.0, most preferably 65.0:35.0 to 95.0:5.0 or 65.0:35.0 to 90.0: 10.0.

It is, for the purpose of this invention, irrelevant by which processes the cis- and trans-2,5-bis(isocyanatomethyl) tetrahydrofuran, are generated, i.e., with or without use of phosgene. Preferred in the industrial production of the 2,5-bis(isocyanatomethyl) tetrahydrofuran is the phosgenation in the liquid-phase or the gas-phase and even more preferred by gas-phase phosgenation as described for example in EP 0 764 633 A2.

The precursor for the 2,5-bis(isocyanatomethyl) tetrahydrofuran of the invention can be obtained from different sources but is preferably from a biobased source.

A further aspect of the invention relates to a method for production of a polyisocyanate composition comprising the steps of:

i. provision of the 2,5-bis(isocyanatomethyl) tetrahydrofuran according to the invention, optionally a catalyst and optionally a further coreactant selected from an alcohol, thiol, amine, water, $CO_2$ or an isocyanate different from 2,5-bis(isocyanatomethyl) tetrahydrofuran and having an NCO functionality of > 1 or any combination of two or more thereof,

ii. conversion of the 2,5-bis(isocyanatomethyl) tetrahydrofuran provided in step i, optionally in presence of the catalyst provided in step i, optionally in presence of a further coreactant selected from an alcohol, thiol, amine, water, $CO_2$ or an isocyanate different from 2,5-bis(isocyanatomethyl) tetrahydrofuran and having an NCO functionality of > 1 or any combination of two or more thereof provided in step i, to obtain the polyisocyanate composition.

This inventive method thus comprises an oligomerization step of a mixture of cis- and trans-2,5-bis(isocyanatomethyl), optionally in the presence of a catalyst or further coreactants, and results in a polyisocyanate composition. Such a modification of TEFUDI can be done according to methods for example described in J. Prakt. Chem. 336 (1994) 185 - 200, in DE-A 1 670 666, DE-A 1 954 093, DE-A 2 414 413, DE-A 2 452 532, DE-A 2 641 380, DE-A 3 700 209, DE-A 3 900 053 and DE-A 3 928 503 or in EP-A 0 336 205, EP-A 0 339 396 EP-A 0 798 299, EP-A 0 962 454, EP-A 0 962 455, EP-A 2 785 760, EP-A 2 883 895, EP-A 3 107 922, EP-A 3 107 948 and EP-A 3 337 836.

Coreactants optionally used for the inventive method and optionally most likely contained in reacted form in the inventive polyisocyanate composition, in addition to the cis- and trans-2,5-bis(isocyanatomethyl) tetrahydrofuran themselves, may be the usually bi- or higher-functional coreactants having Zerevitinoff-active hydrogen that are customary in polyurethane chemistry, for example water, alcohols, thiols and amines. These compounds preferably have an average OH, NH or SH functionality of at least 1.5. These may, for example, be low molecular weight diols (e.g. ethane-1,2-diol, propane-1,3- or -1,2-diol, butane-1,4-diol), triols (e.g., glycerol, trimethylolpropane) and tetraols (e.g., pentaerythritol), short-chain polyamines, but also polyaspartic esters, polythiols and/or polyhydroxy compounds such as polyether polyols, polyester polyols, polyurethane polyols, polysiloxane polyols, polycarbonate polyols, polyether polyamines, polybutadiene polyols, polyacrylate polyols and/or polymethacrylate polyols, and the copolymers thereof.

The optionally comprised further isocyanates having an NCO functionality > 1 in the inventive method are in principle known to those skilled in the art. Preference is given to pentamethylene 1,5-diisocyanate (PDI), hexamethylene 1,6-diisocyanate (HDI), 2-methylpentane 1,5-diisocyanate, 2,4,4-trimethylhexane 1,6-diisocyanate, 2,2,4-trimethylhexane 1,6-diisocyanate, 4-isocyanatomethyloctane 1,8-diisocyanate, 3(4)-isocyanatomethyl-1-methylcyclohexyl isocyanate (IMCI), isophorone diisocyanate (IPDI), 1,3- and 1,4-bis(isocyanatomethyl)benzene (XDI) and/or 1,3- and 1,4-bis(isocyanatomethyl)cyclohexane (H6XDI). Particular preference is given to PDI, HDI, 2,4,4-trimethylhexane 1,6-diisocyanate, 2,2,4-trimethylhexane 1,6-diisocyanate, 4-isocyanatomethyloctane and/or 1,8-diisocyanate, and even more preference is given to PDI and/or HDI.

As catalysts to be optionally used for the NCO-NCO reactions, e.g., to form the isocyanurate, iminooxadiazindione and/or allophanate structures from the cis- and trans-2,5-bis(isocyanatomethyl) tetrahydrofuran, the typical compounds known to be catalytically active to isocyanates are suitable. These catalysts include, in addition to compounds of ionic structure for example with "onium" cations (ammonium, phosphonium, etc.) and nucleophilic anions such as hydroxide, alkanoate, carboxylate, heterocycles having at least one negatively charged nitrogen atom in the ring, especially azolate, imidazolate, triazolate, tetrazolate, fluoride, hydrogendifluoride, higher polyfluorides or mixtures of these (adducts of more than one equivalent of HF onto compounds containing fluoride ions), it being possible for the fluorides, hydrogendifluorides and higher polyfluorides to lead under suitable reaction conditions to products having a higher iminooxadiazinedione group content, also neutral bases such as tertiary amines or phosphanes (phosphines). Especially in the latter case structural variation makes it possible to cover a wide selectivity range; from high uretdione selectivity through to high "trimer" selectivity, the latter typically resulting in mixtures of isocyanurates and iminooxadiazinediones.

The optional catalysts may be used individually or in any desired mixtures of any two or more thereof. For instance, the solutions of quaternary ammonium hydroxides in various alcohols, depending on the pKa value of the base and of the alcohol used, are present partially or completely as ammonium salts with alkoxide anion. This equilibrium can be shifted wholly to the side of complete alkoxide formation by removing the water of reaction resulting from this reaction. Suitable methods for water removal in this case are all methods known from literature for this purpose, in particular (azeotropic) distillation, this optionally being with the aid of a suitable entrainer if the alcohol used as solvent is not suitable as such.

The progress of the reaction in the method of the invention can be monitored by determining the NCO content by titrimetric means as per DIN EN ISO 11909:2007-05. On attainment of the desired NCO content ("degree of polymerization") the reaction is stopped by suitable means depending on the modification reaction and/or the use of a catalyst or not. Preferably catalysts are deactivated by addition of suitable catalyst poisons.

According to a further preferred embodiment, the reaction is taken to a point where the reaction mixture has a degree of oligomerization of 10% to 40%, preferably of 15% to 30%.

"Degree of oligomerization" presently refers to the percentage of the isocyanate groups originally present in the starting mixture that is consumed during the reaction according to the invention. The degree of oligomerization in percent can be calculated according to the following formula:

$$\text{Degree of oligomerization} = (\text{NCO start} - \text{NCO end}) / \text{NCO start} \times 100.$$

The reaction can be discontinued, for example, when the target degree of oligomerization has been reached. This degree of oligomerization is reached generally after a reaction time of 30 minutes to 8 hours, preferably of 1 to 6 hours.

The reaction may be terminated for example by cooling of the reaction mixture to ambient temperature, in particular 20 - 25 °C. In general, however, the reaction is ended by addition of a catalyst poison and optional subsequent brief heating of the reaction mixture to a temperature above 80 °C, for example.

Examples of suitable catalyst poisons are inorganic acids such as hydrochloric acid, phosphorous acid or phosphoric acid, acyl chlorides such as acetyl chloride, benzoyl chloride or isophthaloyl dichloride, sulfonic acids and sulfonic esters, such as methanesulfonic acid, p-toluenesulfonic acid, trifluoromethanesulfonic acid, perfluorobutane sulfonic acid, dodecylbenzenesulfonic acid, methyl and ethyl p-toluenesulfonates, monoalkyl and dialkyl phosphates such as monotridecyl phosphate, dibutyl phosphate, and dioctyl phosphate, and also silylated acids, such as trimethylsilyl methanesulfonate, trimethylsilyl trifluoromethanesulfonate, tris(trimethylsilyl) phosphate, and diethyl trimethylsilyl phosphate.

The amount of catalyst poison needed to end the reaction depends on the amount of catalyst used; in general, an equivalent amount (100 equivalent%) of the catalyst poison is used, based on the catalyst used at the start. Taking into account losses of catalyst potentially occurring during the reaction, 20 to 80 equivalent% of the catalyst poison, based on the amount of catalyst originally used, may also be sufficient to end the reaction.

The stated catalyst poisons may be used either as they are or else in solution in a suitable solvent. If a solvent is employed for dissolving the catalyst poison, preference is given to TEFUDI. The degree of dilution may be selected freely within a very wide range; for example, suitable solutions are those starting from a concentration of ≥25.0% by weight, preferably ≥10.0% by weight.

After the end of reaction, the reaction mixture may be preferably freed from volatile constituents (excess monomeric isocyanate components and any solvents additionally used), preferably by thin-film distillation under reduced pressure, for example at a pressure of below 1.0 mbar, preferably below 0.75 mbar, more preferably below 0.25 mbar, under extremely gentle conditions, as for example at a temperature of 100 to 200 °C, preferably of 120 to 180 °C.

In another embodiment of the method of the invention, said volatile constituents are removed by extraction with suitable solvents that are inert toward isocyanate groups, examples being aliphatic or cycloaliphatic hydrocarbons such as pentane, hexane, heptane, cyclopentane or cyclohexane, from the polyisocyanate composition.

The method of the invention is preferably conducted without solvent. If desired, however, suitable solvents inert toward the reactive groups of the starting components can also be used. Suitable solvents are, for example, the customary paint solvents that are known per se such as ethyl acetate, butyl acetate, ethylene glycol monomethyl or monoethyl ether acetate, 1-methoxyprop-2-yl acetate, 3-methoxy-n-butyl acetate, acetone, 2-butanone, 4-methyl-2-pentanone, cyclohexanone, toluene, xylene, chlorobenzene, white spirit, more highly substituted aromatics, of the kind available commercially, for example, under the names Solventnaphtha, Solvesso®, Isopar®, Nappar®, Varsol® (ExxonMobil Chemical Central Europe, Cologne, Germany) and Shellsol® (Shell 35 Deutschland Oil GmbH, Hamburg, Germany), and also solvents such as propylene glycol diacetate, diethylene glycol dimethyl ether, dipropylene glycol dimethyl ether, diethylene glycol ethyl and butyl ether acetate, N-methylpyrrolidone and N-methylcaprolactam, or any combination of two or more of the aforementioned.

Independent of the optional use of a solvent during the modification reaction of TEFUDI to form the polyisocyanate composition, the inventive method optionally comprises a step of addition of at least one solvent inert towards isocyanate groups to reach a preferred viscosity of <2000 mPas at 23 °C measured according to DIN EN ISO 3219:1994-10. Such optional solvent or mixture of solvents is preferably selected from the aforementioned. In case such an optional step is conducted, a suitable solvent is preferably added in an amount to achieve a solids content of >50% by weight, more preferably a solids content of >80% by weight and most preferably a solids content of >95% by weight.

Another aspect of the invention is a polyisocyanate composition obtainable or obtained, preferably directly obtained, through oligomerization of the inventive 2,5-bis(isocyanatomethyl) tetrahydrofuran or by the inventive method.

In this polyisocyanate composition, oligomers of 2,5-bis(isocyanatomethyl) tetrahydrofuran may comprise isocyanurate, iminooxadiazinedione, allophanate, urethane, urea, uretdione, oxadiazinetrione, carbodiimide, thiourethane, thioallophanate, biuret structures or any combination of two or more of the aforementioned. In accordance with this embodiment, the oligomers of 2,5-bis(isocyanatomethyl) tetrahydrofuran comprise bridging cis- and trans-tetrahydrofuran-2,5-diyl-dimethylene units as structural units which are connected via one exocyclic $CH_2$-moiety to the nitrogen atom of an isocyanate group, to the nitrogen atom of an isocyanurate group, to the nitrogen atom of an iminooxadiazinedione group, to the nitrogen atom of an allophanate group, to the nitrogen atom of a urethane group, to the nitrogen atom of an uretdione group, to the nitrogen atom of an oxadiazinetrione group, to the nitrogen atom of a carbodiimide group, to the nitrogen atom of a thiourethane group, to the nitrogen atom of a thioallophanate group or to the nitrogen atom of a biuret group and which are connected via the other exocyclic $CH_2$-moiety to the nitrogen atom of independently selected any of the before mentioned functional groups. The designation of the two exocyclic $CH_2$-moieties may also be switched. This bridging unit can be detected via standard $^1H$- and/or $^{13}C$-NMR measurements.

In a preferred embodiment of the invention, the polyisocyanate composition further comprises oligomers of a diisocyanate different from cis-2,5-bis(isocyanatomethyl) tetrahydrofuran and trans-2,5-bis(isocyanatomethyl) tetrahydrofuran, for example PDI, HDI, 2-methylpentane 1,5-diisocyanate, 2,4,4-trimethylhexane 1,6-diisocyanate, 2,2,4-trimethylhexane 1,6-diisocyanate, 4-isocyanatomethyloctane 1,8-diisocyanate, 3(4)-isocyanatomethyl-1-methylcyclohexyl isocyanate (IMCI), IPDI, 1,3- and 1,4-bis(isocyanatomethyl)benzene (XDI), 1,3- and 1,4-bis(isocyanatomethyl)cyclohexane (H6XDI), tolylene 2,4- and 2,6-diisocyanate (TDI), bis(4-isocyanatophenyl)methane (4,4'MDI), 4-isocyanatophenyl-2-isocyanatophenylmethane (2,4'MDI) and polycyclic products obtainable by formaldehyde-aniline polycondensation and subsequent conversion of the resulting (poly)amines to the corresponding (poly)isocyanates (polymer MDI).

In a preferred embodiment, the inventive polyisocyanate composition is characterized in that it has an NCO content determined in accordance with DIN EN ISO 11909:2007-05 of from 5.8 to 25.9% by weight, preferably from 7.8 to 24.9% by weight, particularly preferably from 9.7 to 23.9% by weight, based on the total weight of the polyisocyanate composition.

In a preferred embodiment, the inventive polyisocyanate composition is characterized in that it has a residual monomeric 2,5-bis(isocyanatomethyl) tetrahydrofuran content of less than 1.0% by weight, preferably less than 0.5% by weight and more preferably less than 0.1% by weight, based on the total weight of the polyisocyanate composition. The residual monomeric 2,5-bis(isocyanatomethyl) tetrahydrofuran content is determined by gas chromatography in accordance with DIN EN ISO 10283:2007-11 with internal standard.

The invention further relates to a use of the inventive 2,5-bis(isocyanatomethyl) tetrahydrofuran or of the inventive polyisocyanate composition for production of coatings, adhesives or sealants.

A further aspect of the invention relates to a use of the inventive 2,5-bis(isocyanatomethyl) tetrahydrofuran or of the inventive polyisocyanate composition for increasing and/or adjusting the curing time of a polyisocyante composition according to the invention when reacted with an NCO-reactive compound.

The invention further relates to a two-component system containing a component A) comprising the inventive polyisocyanate composition and a component B) comprising at least one NCO-reactive compound.

NCO-reactive compounds of component B) used may be all compounds known to those skilled in the art - including in any desired mixtures with one another - that have an average OH, NH or SH functionality of at least 1.5. These may, for example, be low molecular weight diols (e.g., ethane-1,2-diol, propane-1,3- or -1,2-diol, butane-1,4-diol), triols (e.g., glycerol, trimethylolpropane) and tetraols (e.g., pentaerythritol), short-chain polyamines, but also polyaspartic esters, polythiols and/or polyhydroxy compounds such as polyether polyols, polyester polyols, polyurethane polyols, polysiloxane polyols, polycarbonate polyols, polyether polyamines, polybutadiene polyols and polyacrylate polyols (including, for the purpose of this invention, polyacrylate polyols, polymethacrylate polyols and copolymers thereof).

In a preferred embodiment of the inventive two-component system the at least one NCO-reactive compound is a polyhydroxy compound, preferably a polyether polyol, polyester polyol, polycarbonate polyol and/or polyacrylate polyol.

The inventive two-component system optionally contains auxiliaries and additives, which may for example be cobinders, desiccants, fillers, cosolvents, color or effect pigments, thickeners, matting agents, light stabilizers, coatings additives such as dispersants, thickeners, defoamers and other auxiliaries such as adhesives, fungicides, bactericides, stabilizers or inhibitors and catalysts or emulsifiers which are known to those skilled in the art.

The invention further relates to a one-component system containing

  i. the inventive polyisocyanate composition but no or essentially no NCO-reactive compounds, or

ii. a component A) comprising the inventive polyisocyanate composition, wherein all or essentially all free isocyanate groups have been deactivated with one or more blocking agents, and a component B) comprising at least one NCO-reactive compound.

In the first case, it is for example desired to let the inventive polyisocyanate composition react with a reactant that is available at the site of use, e.g., moisture from a gas phase or a substrate to be coated.

In the second case, the deactivated isocyanate groups or the blocked polyisocyanate composition can be produced, for example, by allowing the polyisocyanate composition to react with a blocking agent. Examples of blocking agents include blocking agents of oxime, phenol, alcohol, imine, amine, carbamic acid, urea, imidazole, imide, mercaptan, active methylene, acid amide (lactam) and bisulfites.

Each of the inventive two-component and one-component system can be used as solvent-borne or waterborne system. In this regard, typical solvents selected from the above-mentioned examples or water can be used.

The invention further relates to an article comprising at least one cured inventive two-component system or cured inventive one-component system; or an article at least partly coated with a cured inventive two-component system or cured one-component system. For the purpose of this invention, the term "article" also comprises a shaped body or coating, each obtainable or produced, preferably directly produced, by curing the inventive two-component system or the inventive one-component system, optionally under the action of heat and/or in the presence of a catalyst, as well as a composite component comprising a material that is joined at least to a shaped body or a coating.

The comparative examples and examples which follow are intended to further illustrate the invention without limiting it.

Examples:

All percentages, unless noted otherwise, are to be understood to mean percent by weight.

All reactions were conducted under a nitrogen atmosphere in glass apparatuses dried beforehand under reduced pressure at 150 - 200 °C.

The stereochemistry of TEFUDI was determined by $^{13}$C NMR spectroscopy. The measurements were conducted on the Bruker DPX 400 or DRX 700 instruments on approx. 50% ($^{13}$C NMR) samples in dry $C_6D_6$, unless noted otherwise, at 100 or 176 MHz ($^{13}$C NMR). The signal of $C_6D_5H$ present in the NMR solvent (7.15 ppm, $^1$H-NMR) or the solvent signal itself (average signal of the 1:1:1 triplet at 128.0 ppm in the $^{13}$C NMR) were used as reference signal.

The NCO content was determined by titration in accordance with DIN EN ISO 10283:2007-11.

Starting materials used

| | |
|---|---|
| TEFUDI cis/trans mixture 99:1 | |
| TEFUDI cis/trans mixture 85:15 | |
| TEFUDI cis/trans mixture 70:30 | |
| HDI | Covestro Deutschland AG |
| IPDI | Covestro Deutschland AG |
| Bis(2-isocyanatoethyl) ether (OBDI) | |
| 1-Butanol | was obtained from Sigma Aldrich |

**Kinetic measurements for urethanization reactions:**

In 250 ml three-neck flasks equipped with a septum for dosing the respective isocyanate, internal temperature control, and a reflux condenser, 1.5 mol of n-butanol was heated to 50 °C under magnetic stirring for each experiment. Subsequently, 0.05 mol of the respective diisocyanate was quickly injected. The internal temperature was maintained at 50 °C by removing the heating bath and cooling with an ice bath if necessary. The NCO content, and thus the conversion, was monitored titrimetrically at regular intervals. The time ($t_{1/2}$) after which only 50% of the originally present NCO groups remained was used to compare the reactivity of the isocyanates used in the inventive examples (4 to 6) as well as in the comparative experiments (1-3 and 7), see Table 1.

**Table 1**. Results of kinetic measurements performed with diisocyanates in the urethanization reaction with n-butanol.

| Example / Diisocyanate | $t_{1/2}$ (s) |
|---|---|
| 1 HDI (non-inventive) | 2080 |
| 2 IPDI (non-inventive) | 3200 |
| 3 $H_{12}$MDI (non-inventive) | 8100 |
| 4 TEFUDI cis/trans (99:1) (inventive) | 717 |
| 5 TEFUDI cis/trans (85:15) (inventive) | 758 |
| 6 TEFUDI cis/trans (70:30) (inventive) | 933 |
| 7 OBDI (non-inventive) | 900 |

Discussion of the results

As can be seen from Table 1, the inventive TEFUDI exhibits much higher reactivity towards NCO-reactive compounds than diisocyanates from the state of the art, such as HDI, PDI or IPDI. It is instead comparable to that of a comparable acyclic diisocyanate. However, unlike in the case of OBDI, reactivity, and thus curing time, can favourably be adjusted based on the ratio of cis- and trans-TEFUDI which provides for a significant advantage over OBDI which exhibits a single non-adjustable reactivity.

**Claims**

1. 2,5-Bis(isocyanatomethyl) tetrahydrofuran consisting of cis-2,5-bis(isocyanatomethyl) tetrahydrofuran and trans-2,5-bis(isocyanatomethyl) tetrahydrofuran in a weight ratio of 1.0:99.0 to 99.9:0.1.

2. 2,5-Bis(isocyanatomethyl) tetrahydrofuran according to claim 1 consisting of cis-2,5-bis(isocyanatomethyl) tetrahydrofuran and trans-2,5-bis(isocyanatomethyl) tetrahydrofuran in a weight ratio of 50.0:50.0 to 99.9:0.1, preferably, 55:45 to 99.9:0.1, more preferably 60.0:40.0 to 99.5:0.5, even more preferably 65.0:35.0 to 99.0: 1.0, most preferably 65.0:35.0 to 90.0: 10.0.

3. Method for production of a polyisocyanate composition comprising the steps of:

   i. provision of the 2,5-bis(isocyanatomethyl) tetrahydrofuran according to claim 1 or 2, optionally a catalyst and optionally a further coreactant selected from an alcohol, thiol, amine, water, $CO_2$ or an isocyanate different from 2,5-bis(isocyanatomethyl) tetrahydrofuran and having an NCO functionality of > 1 or any combination of two or more thereof,
   ii. conversion of the 2,5-bis(isocyanatomethyl) tetrahydrofuran provided in step i, optionally in presence of the catalyst provided in step i, optionally in presence of a further coreactant selected from an alcohol, thiol, amine, water, $CO_2$ or an isocyanate different from 2,5-bis(isocyanatomethyl) tetrahydrofuran and having an NCO functionality of > 1 or any combination of two or more thereof provided in step i, to obtain the polyisocyanate composition.

4. Polyisocyanate composition obtainable or obtained, preferably directly obtained, through oligomerization of the 2,5-bis(isocyanatomethyl) tetrahydrofuran of claim 1 or 2 or by the method of claim 3.

5. Use of the 2,5-bis(isocyanatomethyl) tetrahydrofuran of claim 1 or 2 or of the polyisocyanate composition according to claim 4 for production of coatings, adhesives or sealants.

6. Use of the 2,5-bis(isocyanatomethyl) tetrahydrofuran of claim 1 or 2 or of the polyisocyanate composition according to claim 4 for increasing and/or adjusting the curing time of a polyisocyante composition according to claim 4 when reacted with an NCO-reactive compound.

7. A two-component system containing a component A) comprising the polyisocyanate composition according to claim 4 and a component B) comprising at least one NCO-reactive compound.

8. The two-component system according to claim 7, wherein the at least one NCO-reactive compound is a polyhydroxy compound, preferably a polyether polyol, polyester polyol, polycarbonate polyol and/or polyacrylate polyol.

9. A one-component system containing

   i. the polyisocyanate composition according to claim 4 but no or essentially no NCO-reactive compounds, or
   ii. a component A) comprising the polyisocyanate composition according to claim 4, wherein all or essentially all free isocyanate groups have been deactivated with one or more blocking agents, and a component B) comprising at least one NCO-reactive compound.

10. An article

   i. comprising a cured two-component system according to claims 7 or 8 or a cured one-component system according to claim 9, or
   ii. comprising a substrate and a coating produced from the two-component system according to claims 7 or 8 or the one-component system according to claim 9.

Europäisches
Patentamt

European
Patent Office

Office européen
des brevets

# EUROPEAN SEARCH REPORT

**Application Number**

EP 24 22 1617

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2024/260965 A1 (COVESTRO DEUTSCHLAND AG [DE]) 26 December 2024 (2024-12-26) * page 16, line 16 - page 17, line 16; examples 1,6 * ----- | 1-10 | INV. C08G18/73 C07C263/10 C07C265/04 |
| X | WO 2023/247525 A1 (COVESTRO DEUTSCHLAND AG [DE]) 28 December 2023 (2023-12-28) * page 6, last paragraph; example 7 * * page 11, paragraph 1 * ----- | 1-10 | |
| X,D | GB 936 370 A (MERCK & CO INC) 11 September 1963 (1963-09-11) * page 1, lines 15,16; examples 1,2 * ----- | 1-10 | |
| X | BAI YUNHAN ET AL: "Green and intrinsically safe pathways for the catalytic synthesis of diisocyanates containing the furan ring from 5-hydroxymethylfurfural", GREEN CHEMISTRY, vol. 26, no. 12, 2024, pages 7140-7155, XP093275204, GB ISSN: 1463-9262, DOI: 10.1039/D4GC00546E Retrieved from the Internet: URL:https://pubs.rsc.org/en/content/articlepdf/2024/gc/d4gc00546e> * figure 1 * * page 7143, right-hand column - page 7145, left-hand column * * sentence relating to footnote 12 on page 7141, left column * ----- -/-- | 1-10 | TECHNICAL FIELDS SEARCHED (IPC) C08G C07C |

The present search report has been drawn up for all claims

| Place of search | Date of completion of the search | Examiner |
|---|---|---|
| Munich | 16 May 2025 | Lanz, Sandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................

& : member of the same patent family, corresponding document

EPO FORM 1503 03.82 (P04C01)

page 1 of 3

Europäisches
Patentamt

European
Patent Office

Office européen
des brevets

# EUROPEAN SEARCH REPORT

**Application Number**

EP 24 22 1617

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VARGAS JORGE ANDRÉS ET AL: "Innovations in isocyanate synthesis for a sustainable future", ORGANIC & BIOMOLECULAR CHEMISTRY, vol. 23, no. 3, 11 November 2024 (2024-11-11), pages 487-505, XP093275214, ISSN: 1477-0520, DOI: 10.1039/D4OB01598C Retrieved from the Internet: URL:https://pubs.rsc.org/en/content/articl epdf/2025/ob/d4ob01598c> * scheme 23, footnote 45; page 496, right-hand column - page 497, left-hand column; table 1 * ----- | 1-10 | |
| X | US 2016/096928 A1 (JEOL STEPHANE [FR]) 7 April 2016 (2016-04-07) * formula (III'); claim 2 * * paragraph [0299]; example 1 * * paragraph [0287] * ----- | 1-10 | |
| X | CN 115 232 028 A (UNIV HEBEI TECHNOLOGY) 25 October 2022 (2022-10-25) * claim 4 * ----- -/-- | 4-10 | **TECHNICAL FIELDS SEARCHED (IPC)** |

The present search report has been drawn up for all claims

| Place of search | Date of completion of the search | Examiner |
|---|---|---|
| Munich | 16 May 2025 | Lanz, Sandra |

EPO FORM 1503 03.82 (P04C01)

page 2 of 3

Europäisches
Patentamt

European
Patent Office

Office européen
des brevets

## EUROPEAN SEARCH REPORT

**Application Number**

EP 24 22 1617

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Fitremann Julietlc ET AL: "New C2 Symmetric Bis-Aziridines Efficient Synthesis of (2S,SS)-1,6-Di@-toluenesulfonyloxy)-2,5-Hexanediol, and (2S,3E,5S)-1,6-Di@-toluenesulfonyloxy)-2,5-Hexenediol", Tetrahedron, 1995, pages 9581-9594, XP093275531, Retrieved from the Internet: URL:https://pdf.sciencedirectassets.com/271372/1-s2.0-S0040402000X14007/1-s2.0-0040402095005335E/main.pdf?hash=34beddd37af1c1c6a060f17e3bc08d7b98c7b29007e3af9427c14d0a88300e39&host=68042c943591013ac2b2430a89b270f6af2c76d8dfd086a07176afe7c76c2c61&pii=00404020950053335E&tid=spdf-60821aa5-9390-444f-887c-add69 * scheme 6; compound 17 * | 4-10 | |
| X | SANG HYUP LEE ET AL: "Efficient Synthesis of Medium-Sized Cyclic Ether Diamines", THE JOURNAL OF ORGANIC CHEMISTRY, vol. 67, no. 5, March 2002 (2002-03), pages 1692-1695, XP055328748, United States ISSN: 0022-3263, DOI: 10.1021/jo0108663 * scheme 1; compound 6a * | 4-10 | TECHNICAL FIELDS SEARCHED (IPC) |

The present search report has been drawn up for all claims

| Place of search | Date of completion of the search | Examiner |
|---|---|---|
| Munich | 16 May 2025 | Lanz, Sandra |

EPO FORM 1503 03.82 (P04C01)

## ANNEX TO THE EUROPEAN SEARCH REPORT
## ON EUROPEAN PATENT APPLICATION NO.

EP 24 22 1617

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2025

| Patent document cited in search report | | Publication date | Patent family member(s) | | Publication date |
|---|---|---|---|---|---|
| WO 2024260965 | A1 | 26-12-2024 | EP | 4480979 A1 | 25-12-2024 |
| | | | WO | 2024260965 A1 | 26-12-2024 |
| WO 2023247525 | A1 | 28-12-2023 | CN | 119278196 A | 07-01-2025 |
| | | | EP | 4296260 A1 | 27-12-2023 |
| | | | EP | 4543852 A1 | 30-04-2025 |
| | | | KR | 20250026185 A | 25-02-2025 |
| | | | WO | 2023247525 A1 | 28-12-2023 |
| GB 936370 | A | 11-09-1963 | GB | 936370 A | 11-09-1963 |
| | | | US | 3049552 A | 14-08-1962 |
| US 2016096928 | A1 | 07-04-2016 | CN | 105209519 A | 30-12-2015 |
| | | | EP | 2989144 A1 | 02-03-2016 |
| | | | FR | 3005055 A1 | 31-10-2014 |
| | | | JP | 2016523991 A | 12-08-2016 |
| | | | US | 2016096928 A1 | 07-04-2016 |
| | | | WO | 2014173876 A1 | 30-10-2014 |
| CN 115232028 | A | 25-10-2022 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 936370 A
- WO 2011098272 A2
- WO 2021180709 A
- EP 0764633 A2
- DE 1670666 A
- DE 1954093 A
- DE 2414413 A
- DE 2452532 A
- DE 2641380 A
- DE 3700209 A
- DE 3900053 A
- DE 3928503 A
- EP 0336205 A
- EP 0339396 A
- EP 0798299 A
- EP 0962454 A
- EP 0962455 A
- EP 2785760 A
- EP 2883895 A
- EP 3107922 A
- EP 3107948 A
- EP 3337836 A

**Non-patent literature cited in the description**

- **H. J. LAAS** ; **R. HALPAAP**. *J. Pedain, J. prakt. Chem.*, 1994, vol. 336, 185-200
- *J. Prakt. Chem.*, 1994, vol. 336, 185-200